# EUROPEAN PATENT SPECIFICATION

(11) **EP 2 144 564 B1**
(45) Date of publication and mention of the grant of the patent: **23.07.2014**
(21) Application number: 08737695.0
(22) Date of filing: 02.04.2008
(51) Int. Cl.: A61B 5/16

(54) **DETERMINING STRESS LEVEL BASED ON THE PERFORMANCE OF THE PERSON IN A GAME OR PUZZLE**
DEFINITION DES STRESSNIVEAUS AUF BASIS DER LEISTUNG EINER PERSON BEI EINEM SPIEL ODER PUZZLE
DÉTERMINATION DU NIVEAU DE STRESS SUR LA BASE DE LA PERFORMANCE D'UNE PERSONNE LORS D'UN JEU OU D'UN PUZZLE

(30) Priority: 04.04.2007 EP 07105593
(43) Date of publication of application: 20.01.2010
(73) Proprietor: Koninklijke Philips N.V., 5656 AE Eindhoven (NL)
(72) Inventor: STUT, Wilhelmus, J., J., NL-5656 AE Eindhoven (NL); PELGRIM, Petronella, H., NL-5656 AE Eindhoven (NL); SAINI, Privender, K., NL-5656 AE Eindhoven (NL); VDOVJAK, Richard, NL-5656 AE Eindhoven (NL); WESTERINK, Joanne, H., D., M., NL-5656 AE Eindhoven (NL); VAN LOENEN, Evert, J., NL-5656 AE Eindhoven (NL); WARTENA, Frank, NL-5656 AE Eindhoven (NL)
(74) Representative: Kroeze, Johannes Antonius
(86) International application number: PCT/IB2008/051236
(87) International publication number: WO 2008/122928

(56) References cited:
- US-A- 4 683 891
- US-A- 5 209 494

## Description

### BACKGROUND OF THE INVENTION

### Technical field

The present invention relates to a system, method and software for determining a stress level of a person.

### DESCRIPTION OF RELATED ART

Several surveys show that many people suffer from stress at work. For example, a survey by Northwestern National Life indicates that 40% of workers report that their job is very or extremely stressful. A survey by Yale University indicates that 29% of workers report that they feel quite a bit or extremely stressed at work. According to Anderson et al, high stress yearly generates a cost of $136 per employee, making stress very expensive for employers.

According to the National Institute for Occupational Safety and Health (NIOSH, see http://www.cdc.gov/nlosh/stresswk.html), job stress can be defined as the harmful physical and emotional responses that occur when the requirements of the job do not match the capabilities, resources, or needs of the worker. Job stress can lead to poor health and even injury. Common complaints include loss of concentration, mood and sleep disturbances, upset stomach, and headache.

An important way to reduce the costs of stress and to improve the well-being of people at work is to detect in an early phase whether a person has stress. When detecting stress in an early phase, measures can be taken on time, such as solving stressful working conditions or helping a person to cope with demanding job conditions.

Currently there are many ways to measure stress. A first known approach is to use self-report questionnaires. Still other known methods include interviews, or observations of a person, such as delayed reaction times or heightened tendency to procrastinate. In a more advanced stage the number of mental or physical complaints can be counted in order to determine the severity of these complaints.

According to other known approaches stress is determined by measuring a physiological parameter of a person. For example, stress can be measured by determining the level of the hormone cortisol in the blood. Cortisol is a corticosteroid hormone that is involved in the response to stress.

WO2006/105085 A2 discloses measuring a breathing pattern of a person with a sensor and giving auditory or visual feedback corresponding to the relaxation pattern of the person.

US 4,683,891 A discloses a method of managing and controlling stress in a person engaged in a goal-oriented, cognitive task, which involves simultaneous measurement of a physical parameter of the person which varies in accordance with the level of the person's stress and the productivity of the person performing the task. A programmed computer calculates the stress point for the person beyond which productivity decreases and provides a visual display of the physical parameters and stress point on a cathode ray tube.

The mentioned approaches all have drawbacks for the person-under-test. First of all, he or she must explicitly do a test that is not a natural part of his or her daily life. According to some of the known approaches the person is subjected to tests that only a professional can do. In some cases this means that the person-under-test must even go to a laboratory or a professional infrastructure like a laboratory to be subjected to testing. Furthermore, at the time of performing the tests the person may have suffered from stress for a longer time and complaints may have become severe enough to need actual professional intervention.

It is an object of the invention to provide a system and method for determining the stress of a person that may be integrated in his or her normal routine.

### SUMMARY OF THE INVENTION

This and other objects of the invention are achieved by a system according to claim 1, a method according to claim 14 and a computer program according to claim 15. Favorable embodiments are defined by the dependent claims 2-13 and 16-17.

According to an aspect of the invention a system is provided for determining a stress level of a person based on the performance of the person in a game or puzzle. In a calibration phase the performance of the person in a game or puzzle is measured and information is stored linking the performance to a given stress level of the person when playing the game or puzzle. Subsequently, in an operational phase, the performance of the person in the game or puzzle is measured, again. The stress level of the person is determined based on the measured performance and the information stored in the calibration phase.

The game-based test according to the invention is easier accessible to a person than tests needing professional tools. By integrating the stress test in a pleasant and normal activity like playing a game, people are likely to be less disturbed. For this reason, the test results are likely to better reflect a person's condition. Furthermore, playing a game not only is a means to detect the stress level, but at the same time may help to reduce stress, to relax, to increase concentration, to reduce mental fatigue, and to make the person's memory function better as is described in more detail in the co-pending patent application EP 06124905.8. The invention can be applied in any environment where people suffer from stress. It can be used at home, but also in the office environment in order to detect and reduce stress and to improve the mental health of workers. The game-based test according to the invention preferably is complementary to known tests that need professional tools.

Preferably, in the calibration phase the steps of measuring the performance of the person in the game or puzzle and storing information linking the performance to a given stress level of the person are executed a plurality of times. For example, the calibration phase may last for a month during which the person repeatedly plays games, for example once a day. The system measures the person's performance at each game, and relates this to the given level of experienced stress of the person. In this way the system can calculate the person's average game performance for each experienced stress level.

The system preferably is adapted for receiving an indication from the person of the given stress level in the calibration phase. In this way, the given stress level is obtained in a very easy way without the need of any additional equipment.

Alternatively, the system is adapted for measuring the given stress level in the calibration phase. The given stress level may be measured by measuring a physiological parameter of the person, which is indicative of stress, in a known way. Examples are the body temperature or Galvanic Skin Response. Thereto the system needs to be provided with or connected to suitable sensors.

Preferably, the system is adapted for executing the calibration phase initially and repeating it, periodically. The calibration phase may be repeated every few weeks or months to keep track of learning and experience effects.

According to an embodiment the system is adapted for performing an action towards the person if the determined stress level in the operation phase is high. Since, according to experts short-lived or infrequent episodes of stress pose little risk, preferably the action towards the person is not performed in case of an incidental high stress measurement but only if the determined stress level in the operation phase is high over a period of time. The action towards the person may be the generation of an alert for the person. For example, the system may inform the person of the continued high stress level and it may be suggested to take measures or to contact a professional for further tests or advise. Alternatively, the action is providing distraction to the person. The distraction aims to relax the person. It may be inviting the person to play a different game which is more relaxing than the game that is used for stress determination, to show relaxing pictures or to play relaxing music or to generate certain scents, etc.

For reasons of consistency in the operational phase the system is adapted for inviting the person to play the game or puzzle in time slots with a similar activity level of the person. This is preferably a "low stress" time slot because it is easier to detect a low performance in a "low stress" time slot than in a "high stress" time slot. The time slots with similar activity level may be at a similar time of the day, for example right after lunch.

The system may use known games to determine the stress or special games may be designed for this purpose. According to a preferred embodiment the game is a word memory game comprising neutral words and emotional words. Research has revealed that in a normal, i.e. non-stressed population the emotional words should be recalled better. However, this is not the case for people experiencing stress. They recall fewer emotional words, whereas their performance on recall of neutral words is not deterred. So, the person's performance in this game can be used to determine his/her stress level.

According to an alternative embodiment the game is having the person naming colors in which words are printed. The word list will be chosen to include "neutral" as well as "highly relevant" words. These "highly relevant" words should be in the domain of the "work related stress". In case that the person is stressed the reaction times for naming the color will be longer for the highly relevant words.

According to a further aspect of the invention a method is provided for determining a stress level of a person based on the performance of the person in a game or puzzle, comprising the following steps:
- in a calibration phase: measuring the performance of the person in a game or puzzle and storing information linking the performance to a given stress level of the person when playing the game or puzzle, and
- in an operational phase, measuring the performance of the person in the game or puzzle, again and determining the stress level of the person based on the measured performance and the information stored in the calibration phase.

Preferably, the method according to the invention is implemented by means of a computer program.

These and other aspects of the invention will be apparent from and elucidated with reference to the embodiments described hereinafter.

### BRIEF DESCRIPTION OF THE DRAWINGS

The invention will be better understood and its numerous objects and advantages will become more apparent to those skilled in the art by reference to the following drawings, in conjunction with the accompanying specification, in which:
Figure 1 shows a flow chart of the steps that are executed during the calibration phase in an embodiment of the system according to the invention.
Figure 2 shows a flow chart of the steps that are executed during the operational phase in an embodiment of the system according to the invention.
Figure 3 shows an example of game performance as a function of time.
Figure 4 shows a block diagram of an exemplary embodiment of the system according to the invention.

Throughout the figures like reference numerals refer to like elements.

### DETAILED DESCRIPTION OF THE PRESENT INVENTION

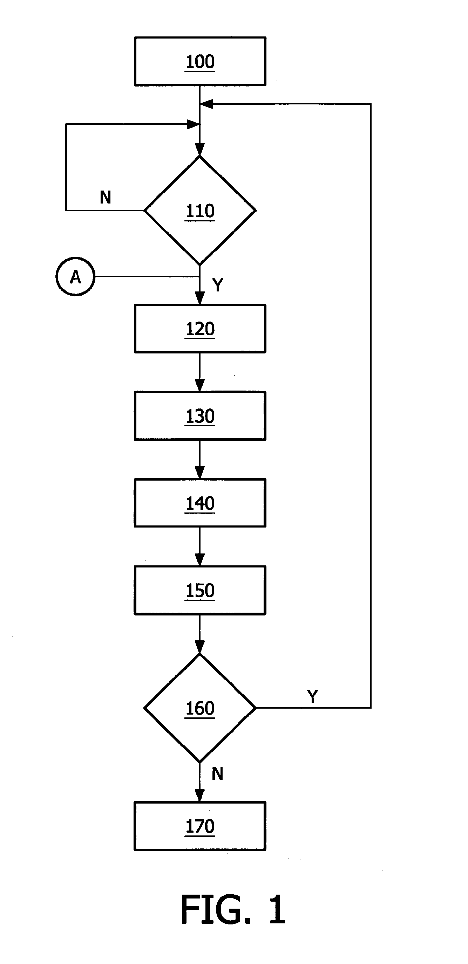

Referring now to figure 1, the steps that are executed during the calibration phase in an embodiment of the system according to the invention will be described.

Before the system can determine a person's stress level via his/her game performance, it should be calibrated. This is due to the fact that every person performs differently under different levels of stress. A possible way to calibrate is to let the person repeatedly play games over a sufficiently long period of time, for example once a day during a month and to let the user indicate the amount of stress experienced before the start of each game. The system measures the person's performance at each game, and relates this to the amount of experienced stress as indicated by the person. In this way the system can calculate the person's average game performance or score for each experienced stress level. For example, the person reaches an average score of 800 points in the "no stress" situation, 600 points at "medium stress", and 400 points at "high stress".

In order to calibrate the system, after the step of initialization 100, it is determined in step 110 if it is time to perform a calibration measurement. If it is not, step 110 is repeated. If it is time for a calibration measurement, in step 120 the person is requested to indicate the amount of stress experienced before starting to play the game. For example, the person may be prompted to indicate if he/she has no stress, medium stress or high stress. Alternatively, the level of stress may be measured by measuring a physiological parameter of the person, which is indicative of stress, in a known way. Examples are the body temperature or Galvanic Skin Response. Thereto the system needs to be provided with or connected to suitable sensors.

Thereafter, in step 130 the person plays the game. In step 140 the game performance or score is determined. To enable the system the measuring of a person's stress level, various game performance indicators may be used. Depending on the game, the following performance indicators are useful (but other indicators may be useful as well):
- the score (i.e. number of points) in a game
- the time needed to complete a game
- the level reached in a game
- the number of errors made in a game
- the number of objects (e.g. balls) that the person can deal with simultaneously
- the speed of objects (e.g. balls) that the person can deal with
- the percentage of correct answers (in e.g. a crossword puzzle)
- the time needed to start/enter/continue a game in a set of alternating games.

These performance indicators can be used both for calibrating the system, and for detecting stress in the subsequent operational phase, that will be described herein after.

In step 150 the measured performance is linked to the stress level that the person indicated in step 120 and both these parameters are stored. In step 160, the system checks if there are more calibration measurements needed. If this is the case the system loops back to step 110. If not, the calibration phase is finished in step 170. In this way, the system builds up a table linking game performance scores and stress levels.

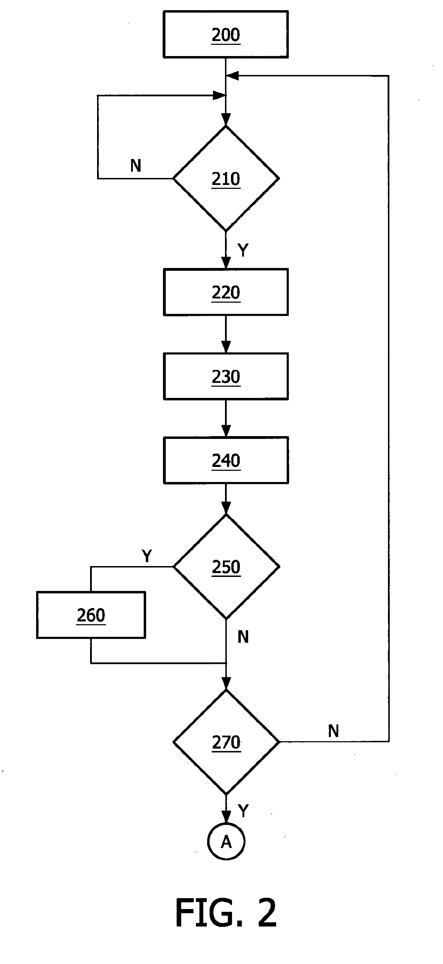

Once the system has been calibrated to a person, it can in a so called operational phase relate the performance of this person in a game session to his/her calculated average score for each experienced stress level. This is shown in figure 2.

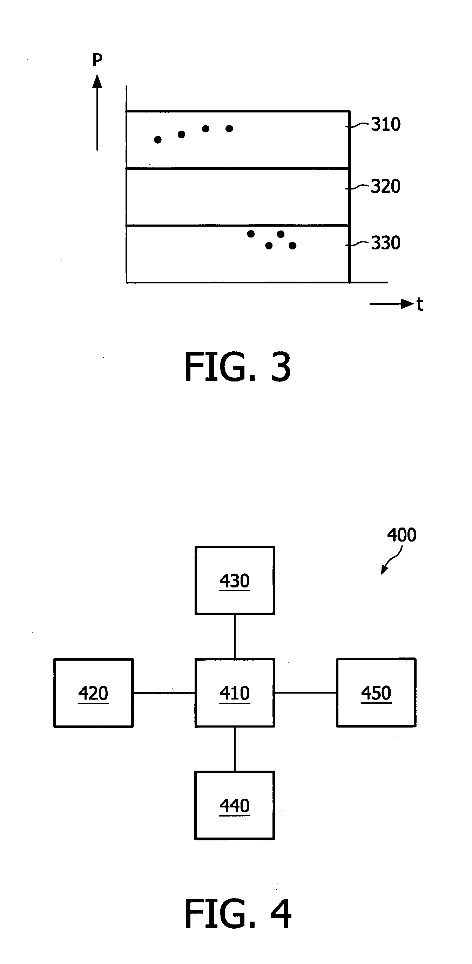

After initialization (step 200) it is determined in step 210 if it is time to determine the stress level of the person. If this is the case, the system invites the person to play the same game that was played during the calibration phase. This game is played by the person in step 220. After the game the person's performance is determined (step 230) and stored (step 240). In step 250 it is determined if an action should be taken towards the person. If the performance stored in step 240 correspond to medium or high stress for a longer period of time (e.g. during a week), the system determines that action should be taken. An example of such a situation is shown in figure 3. Figure 3 shows the performance (P) as a function of time (t). Area 310 (the highest performances) corresponds to scores that were measured during the calibration period when the person indicated that he/she had no stress. Area 320 corresponds to scores that were been measured during the calibration period when the person indicated that he/she had medium stress. Area 330 (the lowest performances) corresponds to scores that were measured during the calibration period when the person indicated that he/she had high stress. Figure 3 shows 8 scores that were measured in the operational phase, i.e. after the system was calibrated. The first 4 scores in the upper area indicate that the person had no stress at these moments. However, the next 4 scores in the lower area suggest that the person had high stress.

In step 260 the system may inform the person about his/her high stress level and may suggest to take measures or to contact a professional for further tests and advise. Note that the system need not inform the person about an incidental performance that may indicate stress. Alternatively or additionally, in step 260 the system may provide distraction to the person. The distraction aims to relax the person. For example, the person may be invited to play a different game which is more relaxing than the game that is used for stress determination, to show relaxing pictures or to play relaxing music or to generate certain scents, as is described in more detail in the co-pending patent application EP 06124905.8.

In step 270 it is determined if calibration measurement(s) should be repeated. If this is the case, steps 120-160 of figure 1 are executed, again. If not, the system loops back to step 210. The calibration should be repeated every few weeks or months in order to keep track of learning and experience effects.

According to experts and the National Institute for Occupational Safety and Health (NIOSH), short-lived or infrequent episodes of stress pose little risk. But when stressful situations go unresolved, the body is kept in a constant state of activation, which increases the rate of wear and tear to biological systems. It therefore appears sufficient to perform the test once a day. For reasons of consistency, it is preferred to play the game at the same moment and preferably in a 'low stress' time-slot. For example, the person can be invited to play the game right after lunch or early in the morning when no meetings are planned. The reason for this is that it is easier to detect a low score in a low-stress time-slot than in a high-stress time-slot.

The person's electronic calendar and/or to do list can be used by the system to determine the best time for both the calibration measurements as well as the measurements during the operational phase. The same calendars can also be used to find high stress times, which can be interrupted by games to avoid build-up of stress.

Any type of game may be used in the system according to the present invention. However, it is preferred to use games derived from known psychological tests for the purpose of measuring stress. These games will be more resistant to factors of boredom or experience. A different type of game may be used to play in order to relax, have fun, and provide a mental break from continued pressure.

Now, two examples will be described of games that are very suitable for the stress determination according to the present invention

Stress causes impairments in memory retrieval. A game can be devised in which people have to learn a list of words that they have to recall later that day or the next day. This list of words can be set up such that it contains neutral as well as emotional words (negative and positive). Neutral words could be words like window, forest, mountain, buildings, kangaroo, etc. So any word that is not related to the stressful situation. In case that the stressful situation is work, emotional words could be: traffic-jam, computer, deadline, presentation, report, meeting, stress, colleagues, lunch, coffee-break, etc. So any word that reasonably is related to work. The human mind protects itself from threats by a number of mechanisms. One of the mechanisms is selective memory. In case of remembering words, humans tend to block the words that arouse negatively, like the emotional words, even when they might be positive like coffee-break or lunch. In a normal population the emotional words should be recalled better. However, this is not the case for people experiencing stress. Fewer emotional words should be recalled, whereas performance on recall of neutral words should not be deterred. See, for a more details on the relationship between stress and memory retrieval "Impaired memory retrieval after psychosocial stress in healthy young men (2005)" by. Kuhlmann, S., Piel, M. and Wolf, O. T. Journal of Neuroscience, 25(11). 2977 - 2982.

Reaction time measurements will reveal whether people are experiencing stress. There are many variants to the Stroop task, and there are a few mechanisms by which it can work to expose people experiencing stress or not. One such mechanism is the attentional bias. A Stroop task could be to have the person name the colors in which words are printed. The word list will be chosen to include "neutral" as well as "highly relevant" words. These "highly relevant" words should be in the domain of the psychopathology, which in the present case is "work related stress". When a person has a certain pathology, words related to that pathology momentarily over-arouse that person. When somebody has overweight and is trying to lose weight, the word 'diet' is going to send a 'rush' through his/her body without that person being able to do anything about it. Similarly, when a person is stressed chronically, there will be certain words that will send a surge through his/her mind and momentarily block him/her from doing anything else, such as naming a color. This can be measured by reaction-times (in milliseconds). So the same emotional (highly relevant) and neutral words as above can be used in this Stroop task. Only in this case the nature of the task will activate a different mechanism. Reaction times for naming the color will be longer for highly relevant (emotional) words. For more information on Stroop tasks, see "The emotional Stroop task and psychopathology" (1996). Williams, J. M. G., Mathews, A., and MacLeod, C. Psychological Bulletin. 120(1), July 1996, 3-24. More variations of the Stroop task can be devised. These tasks can be easily translated into a game-like format.

According to a preferred embodiment of the system the test is implemented on a computer, such as a PC, Xbox, Playstation, mobile phone, PDA, etc. Since the test is personal, the computer should have means such that the person can identify him/herself or should be a personal computer. Figure 4 illustrates an exemplary computer system 400 for implementing the steps according to figure 1. It shows a processor 410, a memory 420, a display 430, an input means 440, which may be a keyboard and a mouse, and communication means 450. In this exemplary embodiment the processor 410 executes instructions stored in the memory 420, for executing the steps shown in figures 1 and 2. The input means 440 are used to control the game. The communication means 450 can be used for exchanging information with external systems (e.g. for adding new games). The display 430 is used to show the game to the person, to inform the person about his/her game performance, and (if needed) suggest the person to take measures or to contact a professional for further tests and advise.

The invention can be resumed as follows: A system and a method for determining a stress level of a person based on the performance of the person in a game or puzzle. First, in a calibration phase the person is asked to judge his/her currently experienced stress level and the person is asked to play a game or puzzle resulting in a certain performance score. Subsequently in this calibration phase, the system links the indicated stress level and the performance score. By repeating these steps a number of times, the system builds a table linking game performance scores and stress levels. The calibration phase will be executed initially and once every while to keep the system calibrated.

Subsequently, in an operational phase, the person is occasionally asked to play the game. Then, based on the current performance score and on the table assembled in the calibration phase, the system determines the current stress level. Depending on the level, and on the period over which the level continues, the system may undertake some action, such as alerting the person or providing distraction.

As will be recognized by those skilled in the art, the innovative concepts described in the present application can be modified and varied over a wide range of applications.

Accordingly, the scope of patented subject matter should not be limited to any of the specific exemplary teachings discussed, but is instead defined by the following claims.

Any reference signs in the claims shall not be construed as limiting the scope thereof.

## Claims

1. System for determining a stress level of a person based on the performance of the person in a game or puzzle, wherein the system comprises:
- a processor (410);
- a memory (420);
- a display (430); and
- an input means (440);
wherein the processor (410) is configured to execute instructions stored in the memory (420) for executing the steps of:
- in a calibration phase, measuring (140) the performance of the person in a game or puzzle that is displayed on the display (430) and controllable with the input means (440), and storing (150) in the memory (420) information linking the performance to a given stress level of the person when playing the game or puzzle, and
- in an operational phase, measuring (230) the performance of the person in the game or puzzle again and determining (250) the stress level of the person based on the measured performance and the information stored in the memory (420) in the calibration phase.

2. System for determining a stress level according to claim 1, wherein the processor (410) is configured to execute instructions stored in the memory (420) for, in the calibration phase, executing the steps of measuring (140) the performance of the person in the game or puzzle and storing (150) information in the memory (420) linking the performance to a given stress level of the person a plurality of times.

3. System for determining a stress level according to claim 1, wherein the input means (440) is adapted for receiving (120) an indication of the person of the given stress level in the calibration phase.

4. System for determining a stress level according to claim 1, wherein the system comprises sensors that are adapted for measuring the given stress level in the calibration phase by measuring a physiological parameter of the person.

5. System for determining a stress level according to claim 1, wherein the processor (410) is configured to execute instructions stored in the memory (420) for executing the calibration phase initially and repeating it, periodically.

6. System for determining a stress level according to claim 1, wherein the processor (410) is configured to execute instructions stored in the memory (420) for performing (260) an action towards the person if the determined stress level in the operation phase is high.

7. System for determining a stress level according to claim 6, wherein the processor (410) is configured to execute instructions stored in the memory (420) for performing (260) the action towards the person if the determined stress level in the operation phase is high over a period of time.

8. System for determining a stress level according to claim 6, wherein the action is the generation of an alert.

9. System for determining a stress level according to claim 6, wherein the action is providing distraction to the person.

10. System for determining a stress level according to claim 1, wherein in the operational phase the processor (410) is configured to execute instructions stored in the memory (420) for inviting the person to play the game or puzzle in time slots with a similar activity level of the person.

11. System for determining a stress level according to claim 10, wherein in the operational phase the processor (410) is configured to execute instructions stored in the memory (420) for inviting the person to play the game or puzzle at a similar time of the day.

12. System for determining a stress level according to claim 1, wherein the game is a word memory game comprising neutral words and emotional words.

13. System for determining a stress level according to claim 1, wherein the game is having the person naming colors in which words are printed.

14. Method for determining a stress level of a person based on the performance of the person in a game or puzzle, comprising the following steps:
- in a calibration phase: measuring (140) the performance of the person in a game or puzzle and storing (150) information linking the performance to a given stress level of the person when playing the game or puzzle, and
- in an operational phase, measuring (230) the performance of the person in the game or puzzle, again and determining (250) the stress level of the person based on the measured performance and the information stored in the calibration phase.

15. A computer program comprising computer program code means adapted to perform the steps of claim 14, when said program is run on a computer.

16. A computer program as claimed in claim 15 embodied on a computer readable medium.

17. A carrier medium carrying the computer program of claim 15.

## Patentansprüche

1. System zur Ermittlung eines Stressniveaus einer Person auf Basis der Leistung der Person bei einem Spiel oder Puzzle, wobei das System umfasst:
- einen Prozessor (410);
- einen Speicher (420);
- ein Display (430); sowie
- ein Eingabemittel (440);
wobei der Prozessor (410) so konfiguriert ist, dass er in dem Speicher (420) gespeicherte Instruktionen ausführt, um die folgenden Schritte auszuführen, wonach:
- in einer Kalibrierungsphase die Leistung der Person bei einem Spiel oder Puzzle, das auf dem Display (430) dargestellt wird und mit dem Eingabemittel (440) steuerbar ist, gemessen wird (140) und in dem Speicher (420) Informationen gespeichert werden, welche die Leistung mit einem bestimmten Stressniveau der Person beim Spielen des Spiels oder Puzzles in Zusammenhang bringen, und
- in einer Betriebsphase die Leistung der Person bei dem Spiel oder Puzzle erneut gemessen (230) und das Stressniveau der Person auf der Grundlage der gemessenen Leistung und der in der Kalibrierungsphase in dem Speicher (420) gespeicherten Informationen ermittelt wird (250).

2. System zur Ermittlung eines Stressniveaus nach Anspruch 1, wobei der Prozessor (410) so konfiguriert ist, dass er in dem Speicher (420) gespeicherte Instruktionen ausführt, um in der Kalibrierungsphase die Schritte des Messens (140) der Leistung der Person bei dem Spiel oder Puzzle sowie des Speicherns (150) von, die Leistung mit einem bestimmten Stressniveau der Person in Zusammenhang bringenden Informationen in dem Speicher (420) mehrere Male auszuführen.

3. System zur Ermittlung eines Stressniveaus nach Anspruch 1, wobei das Eingabemittel (440) so eingerichtet ist, dass es eine Angabe der Person bezüglich des bestimmten Stressniveaus in der Kalibrierungsphase empfängt (120).

4. System zur Ermittlung eines Stressniveaus nach Anspruch 1, wobei das System Sensoren umfasst, die so eingerichtet sind, dass sie das bestimmte Stressniveau in der Kalibrierungsphase durch Messen eines physiologischen Parameters der Person messen.

5. System zur Ermittlung eines Stressniveaus nach Anspruch 1, wobei der Prozessor (410) so konfiguriert ist, dass er in dem Speicher (420) gespeicherte Instruktionen ausführt, um die Kalibrierungsphase zu Beginn auszuführen und diese periodisch zu wiederholen.

6. System zur Ermittlung eines Stressniveaus nach Anspruch 1, wobei der Prozessor (410) so konfiguriert ist, dass er in dem Speicher (420) gespeicherte Instruktionen ausführt, um eine Maßnahme gegenüber der Person zu ergreifen, wenn das in der Betriebsphase ermittelte Stressniveau hoch ist.

7. System zur Ermittlung eines Stressniveaus nach Anspruch 6, wobei der Prozessor (410) so konfiguriert ist, dass er in dem Speicher (420) gespeicherte Instruktionen ausführt, um die Maßnahme gegenüber der Person zu ergreifen, wenn das in der Betriebsphase ermittelte Stressniveau über einen Zeitraum hoch ist.

8. System zur Ermittlung eines Stressniveaus nach Anspruch 6, wobei es sich bei der Maßnahme um die Erzeugung eines Warnsignals handelt.

9. System zur Ermittlung eines Stressniveaus nach Anspruch 6, wobei die Maßnahme für die Person eine Ablenkung vorsieht.

10. System zur Ermittlung eines Stressniveaus nach Anspruch 1, wobei der Prozessor (410) so ausgeführt ist, dass er in der Betriebsphase in dem Speicher (420) gespeicherte Instruktionen ausführt, um die Person dazu einzuladen, das Spiel oder Puzzle in Zeitfenstern bei einem gleichen Aktivitätsniveau der Person zu spielen.

11. System zur Ermittlung eines Stressniveaus nach Anspruch 10, wobei der Prozessor (410) so ausgeführt ist, dass er in der Betriebsphase in dem Speicher (420) gespeicherte Instruktionen ausführt, um die Person dazu einzuladen, das Spiel oder Puzzle zur gleichen Tageszeit zu spielen.

12. System zur Ermittlung eines Stressniveaus nach Anspruch 1, wobei es sich bei dem Spiel um ein Wort-Memory-Spiel mit neutralen Wörtern und emotionalen Wörtern handelt.

13. System zur Ermittlung eines Stressniveaus nach Anspruch 1, wobei die Person in dem Spiel Farben benennt, in denen Wörter gedruckt sind.

14. Verfahren zur Ermittlung eines Stressniveaus einer Person auf Basis der Leistung der Person bei einem Spiel oder Puzzle, wobei das Verfahren die folgenden Schritte umfasst, wonach:
- in einer Kalibrierungsphase die Leistung der Person bei einem Spiel oder Puzzle gemessen (140) wird und Informationen gespeichert (150) werden, die die Leistung mit einem bestimmten Stressniveau der Person beim Spielen des Spiels oder Puzzles in Zusammenhang bringen, und
- in einer Betriebsphase die Leistung der Person bei dem Spiel oder Puzzle erneut gemessen (230) wird und das Stressniveau der Person auf der Grundlage der gemessenen Leistung und der in der Kalibrierungsphase in dem Speicher (420) gespeicherten Informationen ermittelt (250) wird.

15. Computerprogramm mit Computerprogrammcodemitteln, die so eingerichtet sind, dass sie die Schritte nach Anspruch 14 ausführen, wenn das Programm auf einem Computer abläuft.

16. Computerprogramm nach Anspruch 15, das auf einem computerlesbaren Medium dargestellt wird.

17. Trägermedium, welches das Computerprogramm nach Anspruch 15 trägt.

## Revendications

1. Système pour déterminer un niveau de stress d'une personne sur la base de la performance d'une personne lors d'un jeu ou d'un puzzle, le système comprenant :
- un processeur (410) ;
- une mémoire (420) ;
- un affichage (430) ; et
- un moyen d'entrée (440) ;
dans lequel le processeur (410) est configuré pour exécuter des instructions stockées dans la mémoire (420) pour exécuter les étapes suivantes :
- dans une phase d'étalonnage, la mesure (140) de la performance de la personne lors d'un jeu ou d'un puzzle qui est affiché sur l'affichage (430) et contrôlable avec le moyen d'entrée (440), et le stockage (150) dans la mémoire (420) d'informations reliant la performance à un niveau de stress donné de la personne, lorsqu'elle joue au jeu ou au puzzle, et
- dans une phase opérationnelle, une nouvelle mesure (230) de la performance de la personne lors du jeu ou du puzzle et la détermination (250) du niveau de stress de la personne sur la base de la performance mesurée et des informations stockées dans la mémoire (420) au cours de la phase d'étalonnage.

2. Système pour déterminer un niveau de stress selon la revendication 1, dans lequel le processeur (410) est configuré pour exécuter les instructions stockées dans la mémoire (420) pour exécuter, au cours de la phase d'étalonnage, les étapes de mesure (140) de la performance de la personne lors du jeu ou du puzzle et de stockage (150) des informations dans la mémoire (420) reliant la performance à un niveau de stress donné de la personne une pluralité de fois.

3. Système pour déterminer un niveau de stress selon la revendication 1, dans lequel le moyen d'entrée (440) est conçu pour recevoir (120) une indication de la personne ayant le niveau de stress donné au cours de la phase d'étalonnage.

4. Système pour déterminer un niveau de stress selon la revendication 1, le système comprenant des capteurs qui sont conçus pour mesurer le niveau de stress donné au cours de la phase d'étalonnage, en mesurant un paramètre physiologique de la personne.

5. Système pour déterminer un niveau de stress selon la revendication 1, dans lequel le processeur (410) est configuré pour exécuter les instructions stockées dans la mémoire (420), pour exécuter la phase d'étalonnage initialement et la répéter, périodiquement.

6. Système pour déterminer un niveau de stress selon la revendication 1, dans lequel le processeur (410) est configuré pour exécuter des instructions stockées dans la mémoire (420) pour réaliser (260) une action envers la personne si le niveau de stress déterminé au cours de la phase de fonctionnement est élevé.

7. Système pour déterminer un niveau de stress selon la revendication 6, dans lequel le processeur (410) est configuré pour exécuter des instructions stockées dans la mémoire (420) pour réaliser (260) l'action envers la personne si le niveau de stress déterminé au cours de la phase de fonctionnement est élevé sur une période de temps.

8. Système pour déterminer un niveau de stress selon la revendication 6, dans lequel l'action est la génération d'une alerte.

9. Système pour déterminer un niveau de stress selon la revendication 6, dans lequel l'action est la fourniture d'une distraction à la personne.

10. Système pour déterminer un niveau de stress selon la revendication 1, dans lequel, dans la phase opérationnelle, le processeur (410) est configuré pour exécuter des instructions stockées dans la mémoire (420) pour inviter la personne à jouer au jeu ou au puzzle à des intervalles de temps avec un niveau d'activité similaire de la personne.

11. Système pour déterminer un niveau de stress selon la revendication 10, dans lequel, dans la phase opérationnelle, le processeur (410) est configuré pour exécuter des instructions stockées dans la mémoire (420) pour inviter la personne à jouer au jeu ou au puzzle à un moment similaire de la journée.

12. Système pour déterminer un niveau de stress selon la revendication 1, dans lequel le jeu est un jeu de mémoire de mots comprenant des mots neutres et des mots émotionnels.

13. Système pour déterminer un niveau de stress selon la revendication 1, dans lequel, au cours du jeu, la personne est amenée à citer des noms de couleurs dans lesquelles les mots sont imprimés.

14. Procédé pour déterminer un niveau de stress d'une personne sur la base de la performance d'une personne lors d'un jeu ou d'un puzzle, comprenant les étapes suivantes :
- au cours d'une phase d'étalonnage : la mesure (140) de la performance de la personne lors d'un jeu ou d'un puzzle et le stockage (150) d'informations reliant la performance à un niveau de stress donné de la personne lorsqu'elle joue au jeu ou au puzzle, et
- au cours d'une phase opérationnelle, une nouvelle mesure (230) de la performance de la personne lors du jeu ou du puzzle et la détermination (250) du niveau de stress de la personne sur la base de la performance mesurée et des informations stockées au cours de la phase d'étalonnage.

15. Programme informatique comprenant des moyens de code de programme informatique conçus pour réaliser les étapes selon la revendication 14, lorsque ledit programme est exécuté sur un ordinateur.

16. Programme informatique selon la revendication 15 intégré à un support lisible par ordinateur.

17. Support comportant le programme informatique selon la revendication 15.
